# EUROPEAN PATENT SPECIFICATION

(11) **EP 2 319 448 B1**
(45) Date of publication and mention of the grant of the patent: **15.02.2012**
(21) Application number: 10178043.5
(22) Date of filing: 20.09.2006
(51) Int. Cl.: A61B 18/20

(54) **Laser hair removal device**
Vorrichtung zur Haarentfernung mit einem Laser
Dispositif d'épilation au laser

(30) Priority: 21.09.2005 GB 0519252
(43) Date of publication of application: 11.05.2011
(62) Divisional of application: 06779479.2
(73) Proprietor: The Dezac Group Limited, Cheltenham Gloucestershire GL50 1SS (GB)
(72) Inventor: Sohi, Daniel, Barking, Essex IG11 8JX (GB); Wolski, Alexander, Gloucester, GL4 4XG (GB); Bitter, Ahmad, London, W2 6PP (GB); Grant, Tony, Stroud, GL5 4SG (GB)
(74) Representative: Newell, William Joseph

(56) References cited:
- EP-A1- 1 285 600
- US-A- 5 820 625
- US-A1- 2004 167 500
- US-A1- 2004 230 260
- US-B1- 6 572 637

## Description

This invention relates to laser hair removal devices and in particular, but not exclusively, to such devices intended for home usage.

In the past, cosmetic hair removal has been achieved in numerous ways including plucking, electrolysis and laser treatment. In the laser treatment process, light at a suitable wavelength (typically from about 600nm to 900nm) is directed towards the skin such that some of the energy is absorbed by the papilla of the hair follicle, which is damaged by the deposition of energy leading to death of the follicle and subsequent hair removal.

Because of the complexity of these devices and the safety considerations inherent with their use, traditionally laser hair removal treatment has been carried out at specialist clinics or salons rather than at home.

US 6572637 discloses a handheld laser beam projecting probe including a semiconductor laser device controlled by a control circuit that has a timer to control the on time of the semiconductor laser device. An adjuster for adjusting the spacing from the skin may include a micro switch for detecting skin contact.

There is therefore a need for a laser hair removal device with inbuilt safety features so that it can be safely used by the domestic market.

Accordingly, this invention provides a laser hair removal device for being directed towards and/or applied to the skin of a user to expose hair follicles to laser radiation for hair removal, said device comprising a body housing a hair removal laser capable of emitting radiation at a wavelength suitable for hair removal, a control means including a timer for interrupting operation of the device after a preset period, and a sensor for determining at least one of contact with, or proximity to, the skin of a user wherein the control means is further operable upon expiry of the preset period, to allow re-energisation of the laser only after the device has been removed from and reapplied to the skin.

This feature prevents overexposure of the skin which could result in burns and forces the user to remove the laser from the targeted area between uses, thereby giving sufficient time for the area to cool and also making relocation of the device onto the exact same area unlikely. The preset period will depend on a number of factors but typically may be of the order of 4 seconds.

The various functions may be performed by components mounted in an applicator type device which contains at least the majority of the control functionality required for operation. Alternatively, the device may be in the form of a separate base unit which contains the majority of the control circuitry with a separate head containing the laser unit and connected to the base unit by a flexible link.

The invention may be performed in various ways, and two embodiments thereof will now be described by way of example only, reference being made to the accompanying drawings in which:-
Figure 1 is a schematic diagram of a first embodiment of a laser hair removal device in accordance with this invention;
Figure 2 illustrates a second embodiment of the a hair removal device in accordance with the invention; and
Figures 3,4 and 5 are views of a part of the hair removal device shown in Figure 2.

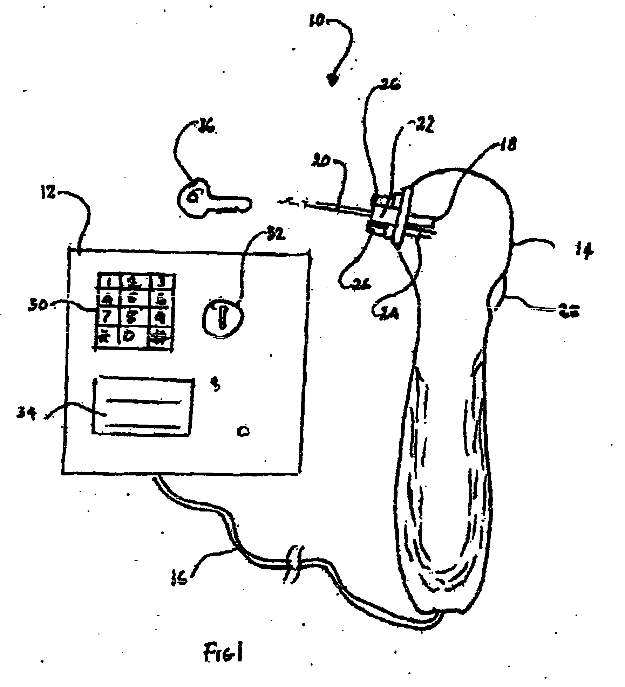

Referring to Figure 1, the hair removal device 10 comprises a base unit 12 and a body in the form of a laser wand 14 connected to the base unit by means of a retractable cord 16. In this particular embodiment the safety control circuitry is contained in the base unit 12 although in other embodiments all or part of this circuitry may be contained in the laser wand 14. The laser wand contains a laser 18 which produces a pulse beam of laser radiation at a wavelength selected to cause hair removal. The laser 18 is arranged to project its beam along a main axis 20. The laser 18 is housed inside the wand 14 and the beam, on leaving the main body of the wand 14 passes through a cylindrical shield 22 of semi-transparent material. Adjacent the laser 18 is a LED 24 designed also to project a high intensity beam of non-injurious visible light along substantially the same main axis 20, passing down the middle of the sleeve 22. To either side of the sleeve are provided two electrodes 26 which are connected to an electrical control circuit which detects when the laser wand 14 is in contact with the skin of a person. This is done by detecting the resistivity of the skin's surface contacted by the electrodes 26. It would be noted that, when the laser wand 14 is pressed against the skin with both contacts 26 in touch with the skin, the laser beam emitted from laser 18 is enclosed by the sleeve 22.

On the back of the wand is an operating switch 28 whose state is determined by the control circuitry in the base unit 12.

On the base unit there is an alphanumeric keypad 30 and a key operated switch 32 together with a display 34. In use, the control circuit in the base unit 12 inhibits operation of the laser 18 until certain conditions are fulfilled. In this particular example, it is necessary firstly to turn the unit on using a key 36 in the key switch 32 and then to punch in PIN code through the keypad 30. When these steps have been carried out, the control unit activates the LED 24 so that a high intensity beam of visible light is projected along the main axis 20 both as a warning of the projected axis of the laser beam from laser 18 when it is activated and also to deter users from pointing it towards their own or others' eyes. Having passed the initial security steps, the wand 14 may be offered up to the area of the skin where hair is to be removed and contact with the skin (as opposed to a non-skin surface) is detected by the control circuit using the electrodes 26. Then, and only then, can the main laser 18 be activated by pressing the operating switch 28.

It will therefore be appreciated that the embodiment illustrated in Figure 1 requires several security stages to be passed before the laser can be operated; the first level security checks confirm the credentials of the user (i.e. being in possession of the right key and code) and thereafter physical security is assessed by ensuring that the device is actually in contact with the skin before the laser is enabled.

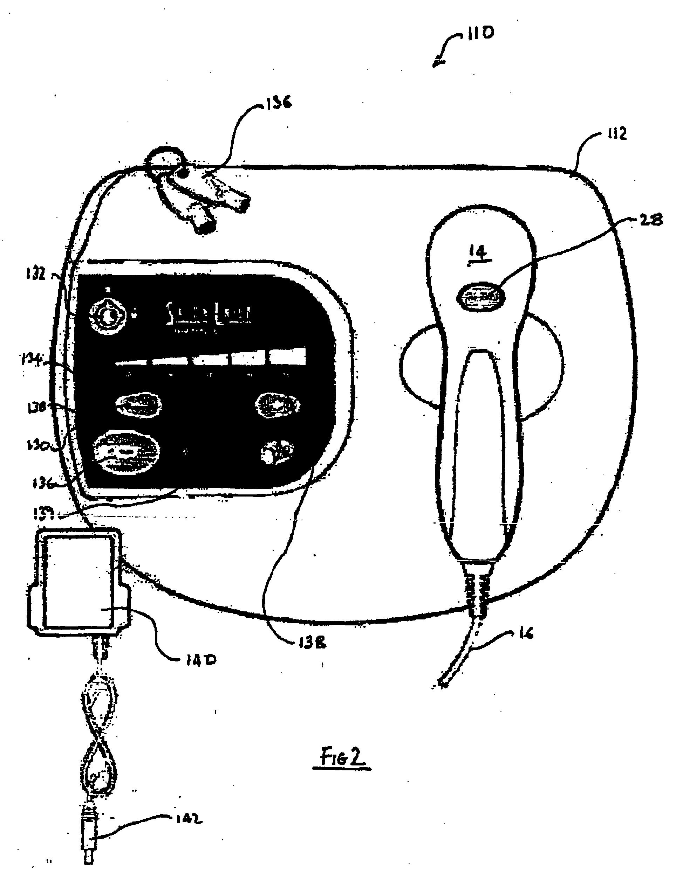

A second embodiment of a hair removal device is illustrated in Figures 2 to 5. Features common to the first and second embodiments have like numbering. The hair removal device 110 has a base unit 112 and a hand held laser wand 14 which operate in a similar manner to the base unit and wand 12,14 described above. In this embodiment the wand 14 rests on, and is held in place by the housing 112. The base unit has a mains adapter power supply 140 which has a low voltage d.c. output plug 142 for fitting into and powering the base unit 112. Alternatively, or as well as, the base unit 122 may be powered by batteries, which may be rechargeable via the mains adapter.

In operation the base unit can be switched on by inserting key 136 into keyswitch 132. As an additional safety precaution, a predetermined series of buttons on the keypad 130 may have to be pressed to enable the laser wand 14 to be powered. The base unit has a power level indicator 134, an enable/hold button 136 , power up/down button 138, and a wand enable warning light 139. The wand 14 has at least the same safety features described above.

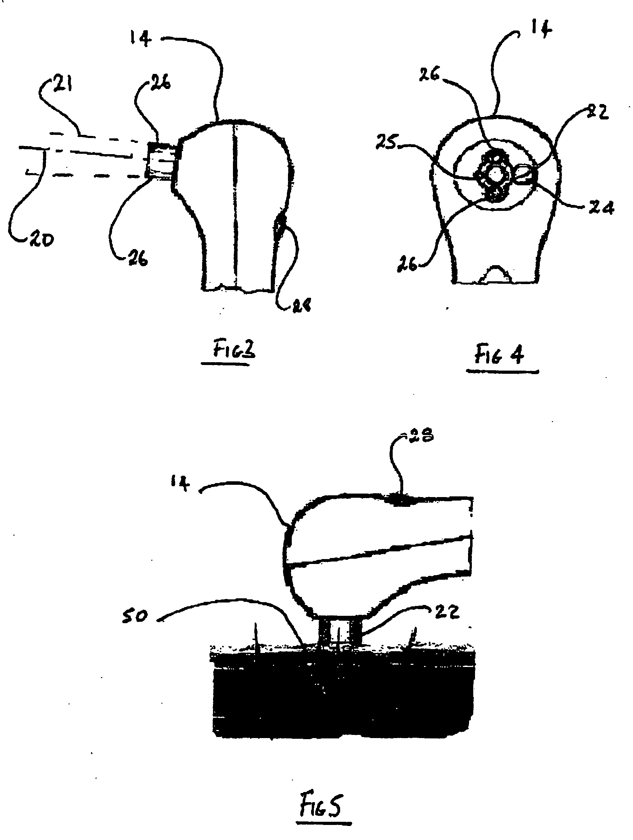

Figures 3,4 and 5 show different views of the head of the wand 14. In Figure 3, electrodes 26 are visible for making contact with, or coming into close proximity with, the skin of a user to enable the wand. If the laser were able to fire, the path of the coherent laser light would pass along axis 20. The beam 21 of high intensity visible but non-injurious light, which is diffuse light, travels along the generally the same axis 20. The diffuse beam 21 is of a lower intensity than the laser beam and is produced when the base 112 is powered or just before the laser operation in order to simulate an eye blinking reflex of any eye in the path of the beam 21.

Figure 4 shows a different view of the head of the wand 14. In this embodiment there is shown a low intensity parallel beam LED 25 which produces a low intensity narrow beam of light along the axis 20. The purpose of this light is to aim the laser so as to guide the user to the hair to be removed. This light can be switched on and can remain on before the laser is operated. A further high intensity LED 24 produces the high intensity light beam 21 for promoting blinking and this light is produced just before the laser is fired.

Figure 5 shows the wand in use aimed, with the aid of the low intensity light from the LED 25. In this position the electrodes 26 are in contact with the skin of a user adjacent body hair 50. The base unit, once activated supplies power to the aim LED 25, to laser activation light is press, causing the high intensity LED 24 to illuminate and then the laser to produce light for a predetermined time period, of sufficient duration to cause damage to the papilla of the hair follicle at hair 50, but not of sufficient duration to cause burning of the skin. Control at base unit 112 prevents firing of the laser again for a period sufficient to allow cooling of the user's skin.

The described device embodies a hair removal device utilising a laser generating pulses of sufficient energy and duration to damage papilla of each hair follicle in the path of the beam, with one or more safety features designed to prevent accidental misuse of the device.

The described device embodies a laser hair removal device for being directed towards and/or applied to the skin of a user to expose hair follicles to laser radiation for hair removal, said device comprising a body housing a hair removal laser capable of emitting radiation at a wavelength suitable for hair removal, characterised by a further light emitting element arranged to emit a high intensity beam of radiation non-injurious to the eye in generally the same direction as said hair removal laser, thereby to deter a user from aiming the device towards their eye.

The high intensity beam is non-injurious in the sense that whilst it might be unpleasant to look into the beam, no permanent injury is caused by that beam.

Preferably the high intensity beam is turned on as soon as the device is powered and before the hair removal laser is actuated. In most instances, the hair removal laser will be fired on demand using a trigger or the like. The laser will typically generate a pulsed beam of laser radiation. The light emitting element preferably emits light at a wavelength to which the eye is particularly sensitive such as light of wavelength of the order of 500-600nm and ideally around 555mn. The light emitting element may comprise an LED designed to provide light at the required frequency.

The described device embodies a laser hair removal device for being direct towards and/or applied to the skin of a user to expose hair follicles to laser radiation for hair removal, said device comprising a body housing a hair removal laser capable of emitting radiation at a wavelength suitable for hair removal, characterised by a filter element adjacent the end of the device to attenuate in use reflection of the beam of the hair removal laser from the skin of the user.

Preferably, the filter element comprises a tubular element extending from the end of the device around the emitted laser beam. The tubular element may conveniently be cylindrical.

The described device embodies a laser hair removal device for being directed towards and/or applied to the skin of a user to expose hair follicles to laser radiation for hair removal, said device comprising a body housing a hair removal laser capable of emitting radiation at a wavelength suitable for hair removal, characterised by a sensor for detecting contact of the device with the skin and/or proximity therewith, and control means responsive to the output of said sensor for inhibiting operation of said removal laser when the device is not in contact with or close to the skin.

The sensor may conveniently comprise two conductive contact elements and an electrical circuit for determining contact thereof with the skin. The electrical circuit may for example determine the electrical resistance. Preferably, the distance of the two contact elements is comparable in size to, or less than the size of, an eye opening to prevent firing directly into the eye. This distance is preferably in the range of about 0.5 to 1.5 cm from the centre of one element to the centre of the other element.

Other sensors could be used with similar function which determine the distance or proximity of the device to the skin. Thus, a light beam or electromagnetic radiation beam may determine the distance of the device from the skin and this output may be used by the control means to inhibit operation.

The described device embodies a laser hair removal device for being directed towards and/or applied to the skin of a user to expose hair follicles to laser radiation for hair removal, said device comprising a body housing a hair removal laser capable of emitting radiation at a wavelength suitable for hair removal, characterised by control means for operating said hair removal laser, said control means including a lock for preventing unauthorised use.

Said lock may take various forms; for example, it could include a keypad for the user to input an identification code, with operation being inhibited unless the correct code is input; it could include a key operated switch such that only a user with the key can turn the device on. Furthermore, the control means may inhibit operation of the laser unless a series of two or more actions are performed. For example, the control means may inhibit operation of the laser until a contact or proximity sensor detects both that the device is safely adjacent the skin and that the user has pressed an activation switch. The control means may require that these actions are performed in the correct order.

## Claims

1. A laser hair removal device (10,110) for being directed towards and/or applied to the skin of a user to expose hair follicles to laser radiation for hair removal, said device comprising:
a body (14) housing a hair removal laser (18) capable of emitting radiation at a wavelength suitable for hair removal,
a control means including a timer for interrupting operation of the device after a preset period, and
a sensor (26) for determining at least one of contact with, or proximity to, the skin of a user,
**characterised in that** the control means is further operable upon expiry of said preset period to allow re-energisation of the laser only after the device has been removed from and reapplied to the skin.

2. A laser hair removal device (10,110) as claimed in Claim 1, wherein the body (14) of the device includes components which contain at least the majority of the control functionality required for operation.

3. A laser hair removal device as claimed in Claim 1, wherein the device is in the form of a separate base unit (12,112) which contains the majority of the control circuitry with a separate wand (14) containing the laser unit and connected to the base unit by a flexible link (16).

## Patentansprüche

1. Laser-Haarentfernungsgerät (10, 110), das gegen die Haut eines Benutzers gerichtet wird und / oder auf der Haut eines Benutzers angewendet wird, um Haarfollikel zur Haarentfernung der Laserstrahlung auszusetzen, umfassend einen Körper (14), der einen Haarentfemungs-Laser (18) aufnimmt, welcher in der Lage ist, eine Strahlung mit einer für die Haarentfernung geeigneten Wellenlänge zu emittieren, ferner umfassend eine Steuervorrichtung mit einem Zeitgeber zur Unterbrechung des Betriebs des Gerätes nach einer vorher eingestellten Zeitspanne, und schließlich umfassend einen Sensor (26) zur Bestimmung wenigstens eines Kontaktes mit der Haut eines Benutzers oder nahe der Haut eines Benutzers, **dadurch gekennzeichnet, daß** die Steuervorrichtung nach dem Ablauf der vorher eingestellten Zeitspanne weiterarbeitet, um den erneuten Laser-Betrieb nur zu ermöglichen, nachdem das Gerät von der Haut entfernt worden ist, und erneut auf der Haut Anwendung findet.

2. Laser-Haarentfernungsgerät (10, 110) nach Anspruch 1, **dadurch gekennzeichnet, daß** der Körper (14) des Gerätes Komponenten aufweist, die zumindest die Mehrzahl der für den Betrieb erforderlichen Steuerfunktionen enthalten.

3. Laser-Haarentfernungsgerät (10, 110) nach Anspruch 1, **dadurch gekennzeichnet, daß** das Gerät die Form einer separaten Basiseinheit (12, 112) hat, welche die Mehrzahl der Steuerschaltungsteile mit einer separaten Wand (14) umschließt, die die Lasereinheit enthält und an die Basiseinheit durch eine flexible Verbindung (16) angeschlossen ist.

## Revendications

1. Dispositif d'épilation au laser (10, 110) destiné à être dirigé vers et/ou appliqué sur la peau d'un utilisateur pour exposer les follicules pileux à un rayonnement laser pour une épilation, ledit dispositif comprenant :
- un corps (14) contenant un laser d'épilation (18) capable d'émettre un rayonnement à une longueur d'onde appropriée pour une épilation,
- un moyen de commande comprenant une minuterie pour interrompre le fonctionnement du dispositif après une période prédéfinie, et
- un capteur (26) pour déterminer au moins l'un d'un contact ou d'une proximité avec la peau d'un utilisateur,
**caractérisé par le fait que** le moyen de commande est en outre actionnable lors de l'expiration de ladite période prédéfinie pour autoriser une nouvelle excitation du laser seulement après que le dispositif a été enlevé de la peau et réappliqué sur la peau.

2. Dispositif d'épilation au laser (10, 110) selon la revendication 1, dans lequel le corps (14) du dispositif comprend des composants qui contiennent au moins la majorité de la fonctionnalité de commande requise pour le fonctionnement.

3. Dispositif d'épilation au laser selon la revendication 1, dans lequel le dispositif se présente sous la forme d'une usité de base séparée (12, 112) qui contient la majorité des circuits de commande avec une tête séparée (14) contentant l'unité laser et reliée à l'unité de base par une liaison souple (16).
